# FASCICULE DE BREVET EUROPEEN

(11) **EP 1 194 238 B1**
(45) Date de publication et mention de la délivrance du brevet: **03.11.2004**
(21) Numéro de dépôt: 00938897.6
(22) Date de dépôt: 08.06.2000
(51) Int. Cl.: B01J 39/12, C22B 3/00, B01J 38/74, C07C 51/31, C07C 51/47

(54) **SEPARATION SELECTIVE DU FER PAR TRAITEMENT AVEC UNE RESINE ECHANGEUSE D'IONS COMPRENANT DES GROUPEMENTS ACIDES DIPHOSPHONIQUES**
SELEKTIVE TRENNUNG VON EISEN MIT EINEM DIPHOSPHONSÄUREGRUPPEN ENTHALTENDEN IONENAUSTAUSCHERHARZ
SELECTIVE SEPARATION OF IRON BY TREATMENT WITH AN ION-EXCHANGING RESIN COMPRISING DIPHOSPHONIC ACID GROUPS

(30) Priorité: 15.06.1999 FR 9907790
(43) Date de publication de la demande: 10.04.2002
(73) Titulaire: Rhodia Polyamide Intermediates, 69192 Saint-Fons (FR)
(72) Inventeur: GOTTELAND, Patrice, F-69006 Lyon (FR); LOGETTE, Sébastien, F-69007 Lyon (FR)
(74) Mandataire: Esson, Jean-Pierre
(86) Numéro de dépôt international: PCT/FR2000/001587
(87) Numéro de publication internationale: WO 2000/076661

(56) Documents cités:
- EP-A- 0 761 636
- CHIARIZIA R ET AL: "Diphonix resin: a review of its properties and applications" SEPARATION SCIENCE AND TECHNOLOGY, vol. 32, no. 1-4, 1997, pages 1-35, XP000878996 NEW YORK,NY, US
- DATABASE WPI Section Ch, Week 197134 Derwent Publications Ltd., London, GB; Class A43, AN 1971-55098S XP002130637 & SU 277 766 A (LUBYANITSKI IY OLKHOVSKAY)

## Description

L'invention concerne un procédé pour séparer sélectivement le fer d'autres ions métalliques notamment des ions présents dans certains catalyseurs d'oxydation.

Elle se rapporte également à un procédé de recyclage des catalyseurs dans la réaction d'oxydation d'alcools et éventuellement cétones en acides carboxyliques et plus particulièrement l'oxydation d'alcools cycliques et cétones cycliques en acides dicarboxyliques tels que l'oxydation du cyclohexanol et/ou cyclohexanone en acide adipique.

Ainsi, il est connu de fabriquer de l'acide adipique par oxydation nitrique d'un mélange de cyclohexanol et cyclohexanone. Cette oxydation est généralement réalisée en présence d'un catalyseur contenant du vanadium et du cuivre.

La solution récupérée après séparation des acides dicarboxyliques et notamment de l'acide adipique est traitée pour permettre le recyclage du catalyseur au niveau de la réaction d'oxydation.

Plusieurs procédés de traitement de cette solution ont été proposés. Par exemple, les métaux contenus dans la solution peuvent être extraits par traitement par des résies échangeuses d'ions. La solution épurée en métaux contient les sous produits de la synthèse de l'acide adipique, à savoir les acides glutarique et succinique. Un tel procédé est par exemple décrit dans le brevet US 3 965 164.

Toutefois, ce procédé ne permet pas une élimination des ions fers provenant notamment de la corrosion des installations. Ainsi, le catalyseur s'enrichit en fer à chaque cycle de recyclage. Cet enrichissement peut diminuer l'efficacité du catalyseur ou également polluer l'acide adipique fabriqué.

Plusieurs procédés ont été proposés pour éliminer au moins partiellement le fer sans perte de vanadium et de cuivre.

D'autres procédés consistent à éluer sélectivement et différentiellement le vanadium et le cuivre de la résine échangeuse d'ions en utilisant des solutions d'acide nitrique de concentration plus ou moins élevée ou des solutions d'élution contenant des acides différents tels que l'acide nitrique ou l'acide phosphonique (SU 690 320, US 3 554 692).

Toutefois, ces procédés ne permettent pas une élimination sélective du fer sans perte significative en vanadium ou cuivre compte tenu du rapport de concentration très faible en fer par rapport aux deux autres ions métalliques.

Pour remédier à ces inconvénients, la demande de brevet européen n° 0 761 636 propose un procédé qui consiste à traiter l'éluat contenant les ions fer, cuivre et vanadium par une seconde résine échangeuse d'ions contenant des groupements aminophosphoriques.

Bien qu'améliorant les procédés antérieurs, ce procédé ne permet pas d'éliminer la majeure partie des ions fer sans perte notamment d'ions vanadium, ce qui est très préjudiciable pour le procédé d'oxydation. En effet, ces résines peuvent avoir une sélectivité satisfaisante en fer par rapport au vanadium uniquement dans des conditions de pH très acide, nettement inférieur à 1, c'est à dire quand les composés métalliques sont contenus dans une solution d'acide nitrique concentrée, par exemple.

Un des buts de la présente invention est de proposer un nouveau procédé permettant de séparer sélectivement le fer d'autres ions métalliques, notamment des ions vanadium et donc de proposer un procédé très performant de recyclage du catalyseur d'oxydation de composés organiques, plus particulièrement d'alcools et/ou de cétones en acides carboxyliques, et encore plus préférentiellement de cyclohexanol et/ou cyclohexanone en acide adipique.

A cet effet, l'invention propose un procédé pour séparer sélectivement le fer contenu dans une solution en présence d'autres ions métalliques dont le vanadium. Ces solutions sont généralement des solutions provenant des procédés d'oxydation d'un composé organique en présence d'un catalyseur à base de vanadium.

Selon la caractéristique de l'invention, la solution contenant lesdits ions métalliques est traitée par une résine échangeuse d'ions comprenant des groupements acide diphosphonique.

Au cours de ce traitement, le fer est fixé par la résine, les ions métalliques comme notamment le cuivre et le vanadium restent dans la solution traitée.

Le procédé de l'invention permet de fixer sur la résine au moins 80 % du fer contenu dans la solution.

Selon une autre caractéristique de l'invention, la solution à traiter contenant les ions métalliques a un pH très faible, avantageusement inférieur à 3 et de préférence inférieur à 2.

Selon une caractéristique préférée de l'invention, la résine échangeuse d'ions comprend également des groupements sulfoniques.

L'utilisation de résines comprenant des groupements acides diphophoniques et éventuellement des groupements sulfoniques permet de manière remarquable de fixer le fer contenu dans une solution, sans fixer d'autres cations métalliques tels que le cuivre et plus particulièrement le vanadium.

Ce procédé s'applique plus particulièrement au recyclage des catalyseurs dans les réactions d'oxydation de composés organiques et plus particulièrement d'alcools et/ou cétones en acides carboxyliques. Un tel procédé d'oxydation constitue également un objet de la présente invention.

En effet, dans ces procédés le fer est souvent un élément contaminant provenant principalement de l'attaque corrosive du matériel par le milieu d'oxydation.

On peut citer comme réactions d'oxydation dans lesquelles le procédé de l'invention permet un recyclage efficace du catalyseur, les réactions d'oxydation utilisant comme agent oxydant un composé choisi dans le groupe comprenant l'oxygène, l'air, les peroxydes, l'eau oxygénée, l'acide nitrique.

Comme exemple de réaction d'oxydation, on peut citer la réaction de déperoxydation de l'hydroxyperoxyde de cyclohexane.

Il s'applique notamment dans le procédé de fabrication d'acide adipique à partir de l'oxydation de cyclohexanol et/ou cyclohexanone, procédé qui est également un objet de la présente invention.

Dans de tels procédés, il est important d'un point de vue économique et pour éviter le rejet de métaux dans l'environnement, de recycler le catalyseur comprenant des éléments métalliques tels que, par exemple, le cuivre et/ou le vanadium, en minimisant les purges ou le flux de catalyseur non recyclé.

Toutefois, pour qu'un tel procédé de recyclage ne perturbe pas la réaction d'oxydation, il est nécessaire d'éviter le recyclage ou la concentration de certains produits ou sous produits qui ont un rôle de poison pour le catalyseur ou qui peuvent polluer les produits fabriqués.

Dans le cas de la fabrication de l'acide adipique par oxydation du cyclohexanol et/ou cyclohexanone par un oxydant tel que l'acide nitrique, la corrosion des installations entraîne, notamment, la présence de fer dans le milieu réactionnel.

En conséquence, il est important pour le fonctionnement du procédé de prévoir une élimination du fer. Cette élimination doit être obtenue sans élimination des autres ions métalliques utilisés comme catalyseur de l'oxydation tels que le cuivre et le vanadium.

Le procédé de l'invention permet de réaliser cette élimination du fer par traitement de la solution contenant les différents ions métalliques récupérée après séparation des composés organiques et notamment du ou des acides carboxyliques formés. Cette élimination du fer est obtenue par traitement de ladite solution par une résine échangeuse d'ions comprenant des groupements acides diphosphoniques et éventuellement des groupements sulfoniques.

Après traitement, la solution comprend les ions métalliques à l'exception des ions fer, ceux-ci étant fixés sur la résine. La quantité fixée est avantageusement supérieure à 80 % de la quantité initialement présente dans la solution.

Ainsi, le procédé de l'invention permet de recycler une solution contenant les ions métalliques utiles pour la catalyse, avec une perte minimale desdits ions, et une absence des ions fer ou une présence de ceux-ci à une concentration très faible et non gênante.

Ainsi, dans le cas de la fabrication de l'acide adipique par oxydation d'un alcool et/ou une cétone par de l'acide nitrique, le traitement de la solution aqueuse contenant les ions métalliques provenant du catalyseur d'oxydation, à savoir préférentiellement du vanadium et du cuivre, par une résine échangeuse d'ions comprenant des groupements diphosphoniques et éventuellement sulfoniques permet une élimination du fer provenant notamment de la corrosion des installations, et le recyclage d'une solution catalytique avec une perte minimale en éléments catalytiques, notamment en vanadium et en cuivre.

En outre, comme la concentration en fer dans le milieu réactionnel est maintenue à un niveau très faible, le procédé de l'invention permet une production d'acide adipique avec une teneur en fer très faible voire nulle.

Le traitement de la solution est réalisé dans un premier mode de réalisation après extraction de l'acide adipique et éventuellement séparation du vanadium précipité. La solution épurée en fer peut être ensuite traitée par une seconde résine échangeuse d'ions qui fixent la totalité des ions métalliques pour éviter un recyclage d'une solution contenant des sous-produits organiques de la réaction d'oxydation tels que l'acide glutarique et/ou l'acide succinique. La solution catalytique recyclée est constituée par la solution d'élution de ladite résine, solution généralement constituée par une solution d'acide nitrique.

Dans un second mode de réalisation, le traitement sur une résine échangeuse d'ions à groupements acides diphosphoniques et éventuellement à groupements sulfoniques peut être effectué sur la solution d'élution de la résine permettant de séparer les sous-produits organiques des ions métalliques et décrites ci avant.

Selon une autre caractéristique de l'invention, la résine échangeuse d'ions à groupements diphosphoniques et éventuellement à groupements sulfoniques est régénérée par élution avec une solution d'acide minérale. Comme acides minéraux convenables, on peut citer l'acide nitrique, l'acide phosphorique, l'acide sulfurique.

De préférence, il est préférable d'utiliser un acide identique à celui utilisé pour réaliser la solution catalytique d'oxydation, généralement l'acide nitrique, pour ainsi éviter une pollution du milieu réactionnel par un autre acide.

Toutefois, il est possible de régénérer la résine avec un acide différent puis de conditionner la résine avec l'acide identique à celui de la solution catalytique, par exemple l'acide nitrique, avant une nouvelle utilisation. Il est également possible de réaliser ce conditionnement par des lavages à l'eau de la résine pour éliminer les traces d'acide d'élution.

Les conditions de mise en oeuvre du traitement sur résine échangeuse d'ions conforme à l'invention sont les conditions classiques d'utilisation des résines. Ainsi la température de mise en oeuvre de ce traitement peut varier de la température ambiante (20°C environ) à une température de 100°C environ, de préférence entre 30°C et 80°C.

De même, la concentration de la solution acide de régénération est classique. Elle peut être, par exemple, comprise entre 10% et 40% en poids.

Les résines échangeuses d'ions conformes à l'invention et comprenant des groupements acides diphosphoniques et éventuellement des groupements sulfoniques sont par exemple, celles décrites dans les brevets US 5 449 462 et 5 281 631.

Ces résines sont obtenues par polymérisation de différents monomères dont certains comprennent des groupements acide diphosphonique. La résine peut être une résine polystyrènique avec des groupements diphosphoniques.

La résine peut également comprendre des groupements carboxyliques et/ou des groupements sulfoniques.

Les procédés de fabrication de ces résines sont décrits dans les deux brevets américains cités ci-dessus. La description de leurs structures est également réalisée dans ces documents.

Ces résines sont notamment commercialisées par la société EICHROM Industries sous le nom commercial EICHROM DIPHONIX ®.

D'autres avantages, détails de l'invention apparaîtront au vu des exemples donnés ci-dessous uniquement à titre indicatif.

### EXEMPLE 1

Dans un récipient, on introduit 600 ml d'une solution aqueuse contenant 1,3 % d'acide nitrique, 10 485 ppm d'ion de cuivre, 1353 ppm d'ion de fer et 281 ppm d'ion de vanadium.

On ajoute 50 ml de résine échangeuse d'ions commercialisée sous la dénomination EICHROM DIPHONIX ®.

Le milieu est mis sous agitation pendant des temps variables.

La concentration en métaux dans la solution est mesurée après des durées variables de mise en contact.

Le graphique 1 représente le pourcentage de métal fixé sur la résine en fonction de la durée de traitement.

De ce graphique, on constate aisément que le fer est fixé presque totalement sur la résine. Au contraire, de faibles quantités de vanadium et de cuivre sont fixées au début de l'opération, ces quantités restant constantes pendant toute l'opération.

Le traitement permet donc une séparation sélective du fer, des autres ions métalliques et notamment du vanadium.

### EXEMPLES COMPARATIFS 2, 3

Des essais similaires ont été réalisés avec des résines échangeuses d'ions comprenant des groupements aminophosphoniques conformément au brevet européen 0 761 636.

Les deux résines utilisées sont respectivement une résine commercialisée sous la dénomination PUROLITE S-940 et une résine commercialisée par la société ROHM & HAAS sous la dénomination commerciale C467.

Les graphiques 2 et 3 représentent le pourcentage d'ions métalliques fixés en fonction de la durée de traitement pour respectivement la résine S940 et C467.

Comme on peut le constater, ces résines fixent au maximum 60 % du fer présent dans la solution, mais surtout fixent presque totalement le vanadium.

En conséquence, ces résines utilisées dans les procédés de recyclage de catalyseurs d'oxydation à base de vanadium présentent un inconvénient majeur.
En outre, elles ne permettent pas de réaliser la séparation sélective du fer par rapport au vanadium.

### EXEMPLES 4, 5c, 6c

L'exemple 1 est répété sur une solution contenant 19,9 % d'acide nitrique, 8875 ppm de cuivre 1192 ppm de fer et 165 ppm de vanadium.

Cette solution correspond à la solution d'éluats par l'acide nitrique d'une résine échangeuse de cations sur laquelle a été traitée une solution provenant d'un procédé d'oxydation de cyclohexanol par de l'acide nitrique.

Les résultats obtenus avec une résine EICHROM DIPHONIX ®, la résine PUROLITE S-940 et la résine RHOM & HAAS C467 sont représentés respectivement par les graphiques 4, 5 et 6.

Comme dans les exemples précédents, la résine conforme à l'invention permet de fixer une quantité importante de fer (au moins 60 % de la quantité initiale) en fixant un minimum des autres cations vanadium et cuivre.

Au contraire, les deux autres résines fixent simultanément au fer une quantité significative de vanadium.

### EXEMPLE 7 - Régénération de la résine EICHROM DIPHONIX ®

Les résines chargées en fer obtenues dans les exemples 1 et 4 sont régénérées par élution avec différentes solutions acides par passage de 100 ml de solution d'élution sur 10 ml de résine.

Les résultats obtenus sont rassemblés dans le tableau suivant. Ces résultats représentent le pourcentage d'ions métalliques récupérés par l'élution par rapport à la quantité fixée.

| | Résine exemple 1 | | | Résine exemple 4 | | |
|---|---|---|---|---|---|---|
| | Cu | Fe | V | Cu | Fe | V |
| HNO₃ | 95 | 29 | 97 | 52 | 14 | 56 |
| H₂SO₄ | 91 | 35 | 95 | 50 | 21 | 53 |
| H₃PO₄ | 87 | 50 | 90 | 45 | 37 | 50 |

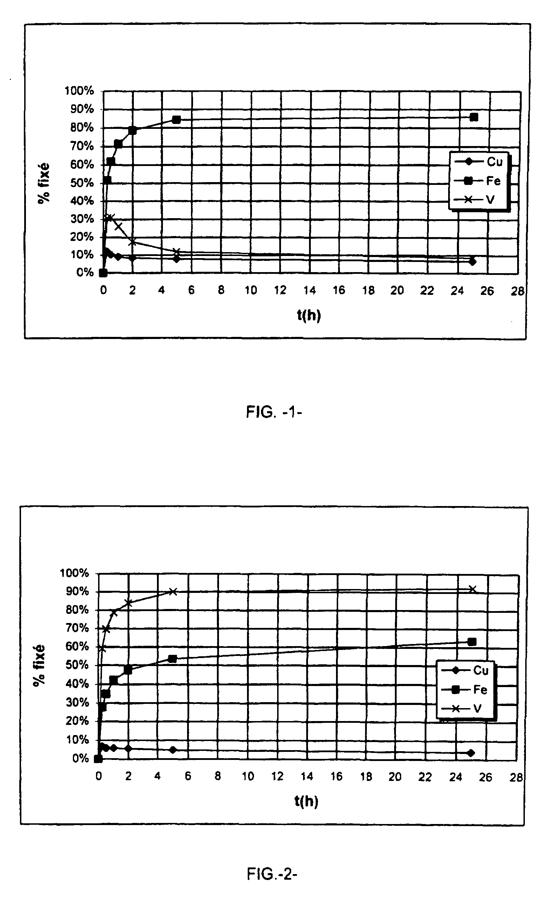

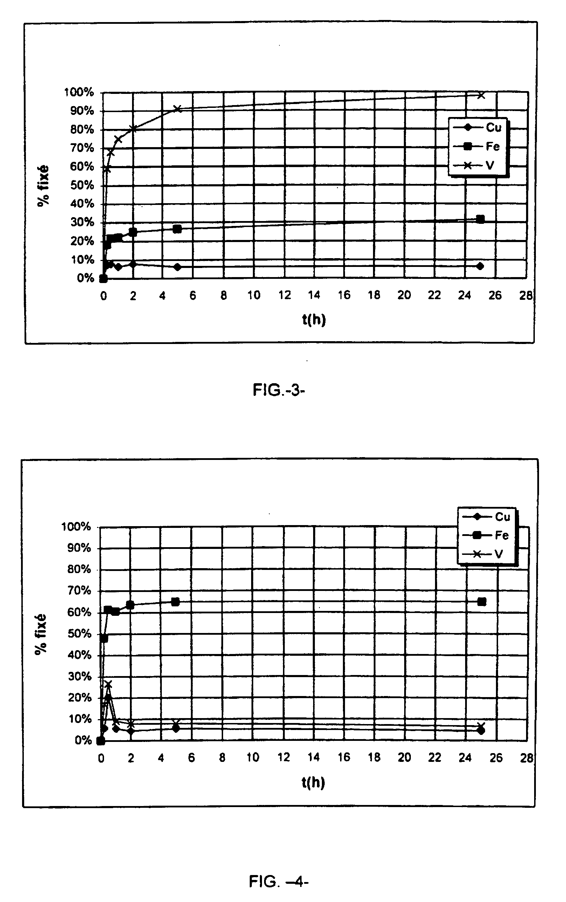

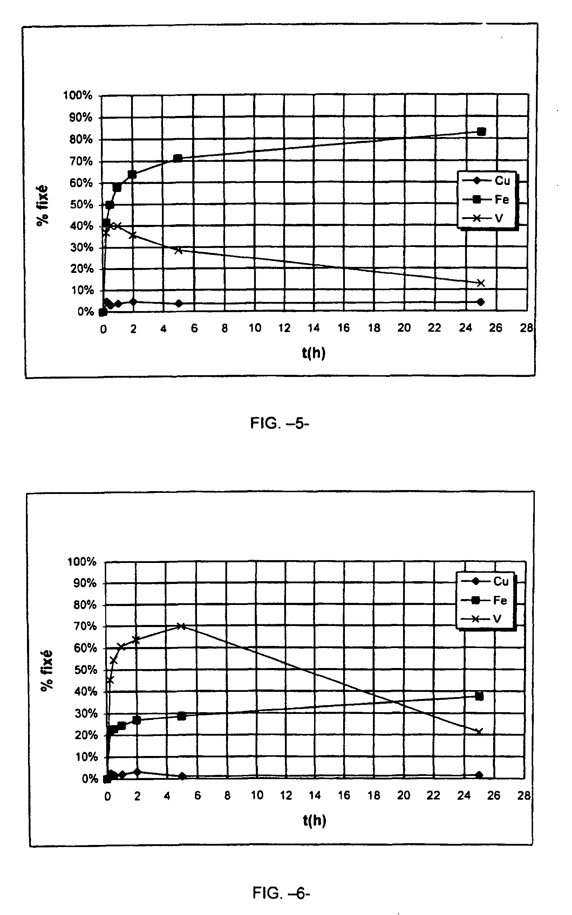

## Revendications

1. Procédé de séparation sélective du fer contenu dans une solution en présence d'autres ions métalliques dont le vanadium, **caractérisé en ce qu'**il consiste à traiter la solution par une résine échangeuse d'ions comprenant des groupements acides diphosphoniques.

2. Procédé selon la revendication 1, **caractérisé en ce que** la résine échangeuse d'ions comprend des groupements sulfoniques.

3. Procédé selon la revendication 1 ou 2, **caractérisé en ce que** la solution contenant les ions métalliques est à un pH inférieur à 3.

4. Procédé selon l'une des revendications précédentes, **caractérisé en ce que** la solution précitée est issue d'un procédé d'oxydation de composés organiques en présence d'un catalyseur.

5. Procédé de recyclage d'un catalyseur dans une réaction d'oxydation d'un composé organique en présence d'un catalyseur comprenant des éléments métalliques, **caractérisé en ce qu'**il consiste à traiter la solution contenant le catalyseur après séparation d'au moins les composés résultant de l'oxydation, par une résine échangeuse d'ions comprenant des groupements acides diphosphoniques pour fixer le fer contenu dans ladite solution, et à recycler ladite solution appauvrie en fer comme solution catalytique de la réaction d'oxydation.

6. Procédé selon la revendication 5, **caractérisé en ce que** la résine échangeuse d'ions comprend des groupes sulfoniques.

7. Procédé selon la revendication 5 ou 6, **caractérisé en ce que** la réaction d'oxydation est mise en oeuvre en utilisant comme agent oxydant un composé choisi dans le groupe comprenant l'oxygène, l'air, les peroxydes, l'eau oxygénée, l'acide nitrique.

8. Procédé selon l'une des revendications 5 à 7, **caractérisé en ce que** la réaction d'oxydation est la réaction d'oxydation d'alcools et/ou cétones en acides carboxyliques.

9. Procédé de fabrication d'acide adipique par oxydation de cyclohexanol et/ou cyclohexanone en présence d'un catalyseur à base d'éléments métalliques, **caractérisé en ce qu'**il consiste à traiter la solution issue de l'oxydation contenant le catalyseur après séparation de l'acide adipique formé, par au moins une résine échangeuse d'ions comprenant des groupements diphosphoniques pour appauvrir ladite solution en ions fer, et à réutiliser ladite solution appauvrie en fer comme catalyseur de la réaction d'oxydation.

10. Procédé selon la revendication 9, **caractérisé en ce que** le catalyseur d'oxydation est à base de cuivre et vanadium.

11. Procédé selon la revendication 9 ou 10, **caractérisé en ce que** la solution contenant le catalyseur est une solution nitrique d'élution d'une résine échangeuse d'ions permettant de séparer les ions métalliques des acides carboxyliques sous-produits de la réaction d'oxydation du cyclohexanol et/ou cyclohexanone en acide adipique.

12. Procédé selon l'une des revendications 9 à 11, **caractérisé en ce que** la résine échangeuse d'ions comprenant des groupements acide diphosphonique est régénérée par une solution acide.

13. Procédé selon la revendication 12, **caractérisé en ce que** la régénération de la résine est réalisée avec un acide différent de l'acide nitrique, ladite résine régénérée est conditionnée par une solution d'acide nitrique ou par lavage à l'eau avant une nouvelle utilisation.

## Patentansprüche

1. Verfahren zur selektiven Abtrennung von Eisen, das in einer Lösung in Anwesenheit von anderen Metallionen, unter ihnen Vanadium, enthalten ist, **dadurch gekennzeichnet, daß** es darin besteht, die Lösung mit einem Ionenaustauscherharz zu behandeln, das Diphosphonsäuregruppen umfaßt.

2. Verfahren nach Anspruch 1, **dadurch gekennzeichnet, daß** das Ionenaustauscherharz Sulfongruppen umfaßt.

3. Verfahren nach Anspruch 1 oder 2, **dadurch gekennzeichnet, daß** die die Metallionen enthaltende Lösung einen pH-Wert von unter 3 besitzt.

4. Verfahren nach einem der vorstehenden Ansprüche, **dadurch gekennzeichnet, daß** die obengenannte Lösung von einem Verfahren zur Oxidation von organischen Verbindungen in Anwesenheit eines Katalysators stammt.

5. Verfahren zum Recycling eines Katalysators in einer Reaktion zur Oxidation einer organischen Verbindung in Anwesenheit eines Katalysators, der metallische Elemente umfaßt, **dadurch gekennzeichnet, daß** es darin besteht, die den Katalysator enthaltende Lösung nach der Abtrennung von mindestens den aus der Oxidation stammenden Verbindungen mit einem Ionenaustauscherharz zu behandeln, das Diphosphonsäuregruppen umfaßt, um das in der genannten Lösung enthaltende Eisen zu fixieren und die genannte, an Eisen abgereicherte Lösung als katalytische Lösung der Oxidationsreaktion zurückzuführen.

6. Verfahren nach Anspruch 5, **dadurch gekennzeichnet, daß** das Ionenaustauscherharz Sulfongruppen umfaßt.

7. Verfahren nach Anspruch 5 oder 6, **dadurch gekennzeichnet, daß** die Oxidationsreaktion unter Verwendung einer Verbindung als Oxidationsmittel durchgeführt wird, die gewählt wird aus der Gruppe, die Sauerstoff, Luft, die Peroxide, Wasserstoffperoxid und Salpetersäure umfaßt.

8. Verfahren nach einem der Ansprüche 5 bis 7, **dadurch gekennzeichnet, daß** die Oxidationsreaktion die Reaktion der Oxidation von Alkoholen und/oder Ketonen zu Carbonsäuren ist.

9. Verfahren zur Herstellung von Adipinsäure durch Oxidation von Cyclohexanol und/oder Cyclohexanon in Anwesenheit eines Katalysators auf der Basis von metallischen Elementen, **dadurch gekennzeichnet, daß** es darin besteht, die aus der Oxidation stammende, den Katalysator enthaltende Lösung nach Abtrennung der gebildeten Adipinsäure durch mindestens ein Ionenaustauscherharz zu behandeln, das Diphosphonsäuregruppen umfaßt, um die genannte Lösung an Eisenionen abzureichern, und die genannte, an Eisen abgereicherte Lösung als Katalysator der Oxidationsreaktion wiederzuverwenden.

10. Verfahren nach Anspruch 9, **dadurch gekennzeichnet, daß** der Oxidationskatalysator auf der Basis von Kupfer und Vanadium besteht.

11. Verfahren nach Anspruch 9 oder 10, **dadurch gekennzeichnet, daß** die den Katalysator enthaltende Lösung eine Salpetersäure-Elutionslösung von einem Ionenaustauscherharz ist, die ermöglicht, die Metallionen der Carbonsäuren als Nebenprodukte der Oxidationsreaktion von Cyclohexanol und/oder Cyclohexanon zu Adipinsäure abzutrennen.

12. Verfahren nach einem der Ansprüche 9 bis 11, **dadurch gekennzeichnet, daß** das Ionenaustauscherharz, das Diphosphonsäuregruppen umfaßt, durch eine saure Lösung regeneriert wird.

13. Verfahren nach Anspruch 12, **dadurch gekennzeichnet, daß** die Regenerierung des Harzes mit einer von Salpetersäure unterschiedlichen Säure realisiert wird, wobei das genannte regenerierte Harz durch eine Lösung von Salpetersäure oder durch Waschen mit Wasser vor einer neuen Verwendung konditioniert wird.

## Claims

1. Process for the selective separation of iron present in a solution in the presence of other metal ions, including vanadium, **characterized in that** it consists in treating the solution with an ion-exchange resin comprising diphosphonic acid groups.

2. Process according to claim 1, **characterized in that** the ion-exchange resin comprises sulphonic groups.

3. Process according to claim 1 or 2, **characterized in that** the solution comprising the metal ions is at a pH of less than 3.

4. Process according to one of the preceding claims, **characterized in that** the abovementioned solution results from a process for the oxidation of organic compounds in the presence of a catalyst.

5. Process for the recycling of a catalyst in a reaction for the oxidation of an organic compound in the presence of a catalyst comprising metal elements, **characterized in that** it consists in treating the solution comprising the catalyst, after separation of at least the compounds resulting from the oxidation, with an ion-exchange resin comprising diphosphonic acid groups, in order to fix the iron present in the said solution, and in recycling the said solution, depleted in iron, as catalytic solution for the oxidation reaction.

6. Process according to claim 5, **characterized in that** the ion-exchange resin comprises sulphonic groups.

7. Process according to claim 5 or 6, **characterized in that** the oxidation reaction is carried out while using, as oxidizing agent, a compound chosen from the group consisting of oxygen, air, peroxides, aqueous hydrogen peroxide solution and nitric acid.

8. Process according to one of claims 5 to 7, **characterized in that** the oxidation reaction is the reaction for the oxidation of alcohols and/or ketones to carboxylic acids.

9. Process for the manufacture of adipic acid by oxidation of cyclohexanol and/or cyclohexanone in the presence of a catalyst based on metal elements, **characterized in that** it consists in treating the solution resulting from the oxidation comprising the catalyst, after separation of the adipic acid formed, with at least one ion-exchange resin comprising diphosphonic groups, in order to deplete the said solution in iron ions, and in reusing the said solution, depleted in iron, as catalyst for the oxidation reaction.

10. Process according to claim 9, **characterized in that** the oxidation catalyst is based on copper and vanadium.

11. Process according to claim 9 or 10, **characterized in that** the solution comprising the catalyst is a nitric solution from the elution of an ion-exchange resin which makes it possible to separate the metal ions from the carboxylic acid byproducts from the reaction for the oxidation of cyclohexanol and/or cyclohexanone to adipic acid.

12. Process according to one of claims 9 to 11, **characterized in that** the ion-exchange resin comprising diphosphonic acid groups is regenerated with an acidic solution.

13. Process according to claim 12, **characterized in that** the regeneration of the resin is carried out with a different acid from nitric acid and the said regenerated resin is conditioned with a nitric acid solution or by washing with water before a fresh use.
